# EUROPEAN PATENT APPLICATION

(11) **EP 4 588 905 A1**
(43) Date of publication of application: **23.07.2025**
(21) Application number: 23894317.9
(22) Date of filing: 19.10.2023
(51) Int. Cl.: C07C 1/12, B01J 23/78, B01J 37/02, B01J 37/03, B01J 37/08, B01J 37/34, C07B 61/00, C07C 9/14, C10G 2/00

(54) **CARBON DIOXIDE REDUCTION CATALYST DEVICE, CARBON DIOXIDE REDUCTION METHOD AND METHOD FOR PRODUCING CATALYST**

(30) Priority: 22.11.2022 JP 2022186448
(71) Applicant: HONDA MOTOR CO., LTD., Tokyo 105-8404 (JP)
(72) Inventor: YAMAMOTO, Osami, Wako-shi, Saitama 351-0193 (JP); SUMI, Hideaki, Wako-shi, Saitama 351-0193 (JP); KAIDA, Chiharu, Wako-shi, Saitama 351-0193 (JP); TSUBAKI, Noritatsu, Toyama-shi, Toyama 930-8555 (JP); HE, Yingluo, Toyama-shi, Toyama 930-8555 (JP)
(74) Representative: Weickmann & Weickmann PartmbB
(86) International application number: PCT/JP2023/037839
(87) International publication number: WO 2024/111305

(57) **Abstract**

The present invention addresses the problem of providing a carbon dioxide reduction catalyst device which is capable of favorably producing a hydrocarbon having 8 to 16 carbon atoms by a hydrogenation reaction of carbon dioxide. In order to solve the problem, the present invention provides a carbon dioxide reduction catalyst device which produces a hydrocarbon by causing a hydrogenation reaction of carbon dioxide and reducing the carbon dioxide, and which comprises a first catalyst that contains, as catalyst metals, Fe and at least one of Ga and Zr, and a second catalyst that contains, as catalyst metals, Fe and Co. With respect to this carbon dioxide reduction catalyst device, the second catalyst is disposed in the downstream of the first catalyst.

## Description

### TECHNICAL FIELD

The present invention relates to a carbon dioxide reduction catalyst device, a carbon dioxide reduction method, and a method for producing a catalyst.

### BACKGROUND ART

Conventionally, efforts for alleviating climate change or mitigating the influence of climate change have been continuing, and in order to achieve this, exhaust gas regulation of automobiles has been further advanced. In particular, there is a need to reduce the amount of carbon dioxide emissions contained in the exhaust gas of internal combustion engines.

In recent years, there has been known a technique for producing a fuel by hydrogenating carbon dioxide. For example, as a catalyst for synthesizing methanol from a mixed gas of carbon dioxide and hydrogen, a catalyst composed of Cu, Zn, and alumina has been proposed (see Patent Document 1).

A fuel obtained by subjecting carbon dioxide to a hydrogenation reaction is required to be able to produce a hydrocarbon having, for example, 5 or more carbon atoms, which can be used as a liquid fuel. As such a technique, a method of preparing a highly branched product having 5 or more carbon atoms by using potassium as a co-catalyst together with an Fe catalyst in an FT (Fischer-Tropsch) synthesis reaction has been proposed (see Patent Document 2).

### Citation List

### Patent Document

Patent Document 1: Japanese Examined Patent Application, Publication No. S45-16682
Patent Document 2: Japanese Unexamined Patent Application (Translation of PCT Application), Publication No. 2005-537340

### DISCLOSURE OF THE INVENTION

### Problems to be Solved by the Invention

Meanwhile, in an effort to reduce an emission amount of carbon dioxide, a technique relating to SAF (Sustainable Aviation Fuel), which is an aviation fuel produced from a biomass-derived raw material, waste, or the like, has attracted attention. It is preferred if the SAF can be produced directly from carbon dioxide using a technique related to the hydrogenation reaction of carbon dioxide. However, the hydrocarbon produced by the technique disclosed in Patent Document 2 has a problem that the production ratio of a hydrocarbon having about 8 to 16 carbon atoms, which is a main component of SAF, is low.

The present invention has been made in view of the above, and an object of the present invention is to provide a carbon dioxide reduction catalyst device capable of preferably producing a hydrocarbon having 8 to 16 carbon atoms by a hydrogenation reaction of carbon dioxide.

### Means for Solving the Problems

(1) The present invention relates to a carbon dioxide reduction catalyst device for reducing carbon dioxide by a hydrogenation reaction of carbon dioxide to produce hydrocarbons, including: a first catalyst containing Fe and at least one of Ga or Zr as catalytic metals; and a second catalyst containing Fe and Co as catalytic metals, in which the second catalyst is disposed downstream of the first catalyst.
(2) The carbon dioxide reduction catalyst device as described in (1), in which at least one of the first catalyst or the second catalyst further contains Na as a catalytic metal.
(3) The carbon dioxide reduction catalyst device as described in (2), in which at least one of the first catalyst or the second catalyst contains Na in an amount that accounts for 0.5 to 1.5 mass% of the catalytic metals.
(4) The carbon dioxide reduction catalyst device as described in any one of (1) to (3), in which the second catalyst contains Co in an amount that accounts for 10 to 40 mass% of the catalytic metals.
(5) The carbon dioxide reduction catalyst device as described in any one of (1) to (4), including a water trap between the first catalyst and the second catalyst.
(6) The carbon dioxide reduction catalyst device as described in any one of (1) to (5), in which the first catalyst contains at least one of an Fe-Ga composite oxide containing Fe and Ga or an Fe-Zr composite oxide containing Fe and Zr.
(7) The present invention also relates to a method of reducing carbon dioxide, the method including using the carbon dioxide reduction catalyst device as described in any one of (1) to (6), in which a catalyst temperature T2 of the second catalyst is lower than a catalyst temperature T1 of the first catalyst.
(8) The carbon dioxide reduction method as described in (7), in which the catalyst temperature T2 is in a range of 260°C to 340°C.
(9) The present invention also relates to a method for producing the first catalyst as described in (1), including a coprecipitation step of extracting a precipitate by a coprecipitation method from an aqueous solution in which a predetermined amount of at least one of a nitrate of Fe, a nitrate of Ga, or a nitrate of Zr is dissolved in distilled water.
(10) The method for producing a catalyst as described in (9), further including, after the coprecipitation step, an impregnation step of adding an aqueous solution containing Na to the precipitate dropwise, drying the solution for a predetermined period of time, and calcining the obtained powder at a predetermined temperature.
(11) The method for producing a catalyst as described in (9) or (10), in which in the coprecipitation step, a precipitation solution is obtained by adding an aqueous urea solution dropwise to an aqueous solution in which a predetermined amount of at least one of the nitrate of Fe, the nitrate of Ga, or the nitrate of Zr is dissolved in distilled water.

### Effects of the Invention

According to the present invention, it is possible to provide a carbon dioxide reduction catalyst device capable of preferably producing a hydrocarbon having 8 to 16 carbon atoms by a hydrogenation reaction of carbon dioxide.

### BRIEF DESCRIPTION OF THE DRAWINGS

[FIG. 1] FIG. 1 is a diagram showing a configuration of a carbon dioxide reduction catalyst device as described in a first embodiment (Example 1).
[FIG. 2A] FIG. 2A is a diagram showing a configuration of a carbon dioxide reduction catalyst device as described in a second embodiment (Example 2).
[FIG. 2B] FIG. 2B is a diagram showing a configuration of a carbon dioxide reduction catalyst device as described in a Comparative Example.
[FIG. 2C] FIG. 2C is a diagram showing a configuration of a carbon dioxide reduction catalyst device as described in a Comparative Example.
[FIG. 3] FIG. 3 is a graph comparing C₈ to C₁₆ yields of the carbon dioxide reduction catalyst devices of Examples and Comparative Examples.
[FIG. 4A] FIG. 4A is a graph showing a relationship between a number of carbon atoms and selectivity in the carbon dioxide reduction catalyst device of Comparative Example 1.
[FIG. 4B] FIG. 4B is a graph showing a relationship between a number of carbon atoms and selectivity in the carbon dioxide reduction catalyst device of Comparative Example 2.
[FIG. 4C] FIG. 4C is a graph showing a relationship between a number of carbon atoms and selectivity in the carbon dioxide reduction catalyst device of Example 1.
[FIG. 5] FIG. 5 is a chart showing C₈ to C₁₆ selectivity and CO₂ conversion of each of the Examples and the Comparative Examples and a relationship with C₈ to C₁₆ yields.
[FIG. 6A] FIG. 6A is a graph showing a relationship between an Na addition amount and a CO₂ conversion in the Examples and the Comparative Examples.
[FIG. 6B] FIG. 6B is a graph showing a relationship between a Na addition amount and C₈-C₁₆ selectivity in the Examples and the Comparative Examples.
[FIG. 6C] FIG. 6C is a graph showing a relationship between a Na addition amount and a C₈-C₁₆ production ratio in the Examples and the Comparative Examples.
[FIG. 7A] FIG. 7A is a graph showing a relationship between a Co addition amount and a CO₂ conversion in the Examples and the Comparative Examples.
[FIG. 7B] FIG. 7B is a graph showing a relationship between a Co addition amount and C₈-C₁₆ selectivity in the Examples and the Comparative Examples.
[FIG. 7C] FIG. 7C is a graph showing a relationship between a Co addition amount and a C₈-C₁₆ production ratio in the Examples and the Comparative Examples.

### PREFERRED MODE FOR CARRYING OUT THE INVENTION

### <Carbon Dioxide Reduction Catalyst Device>

### <<First Embodiment>>

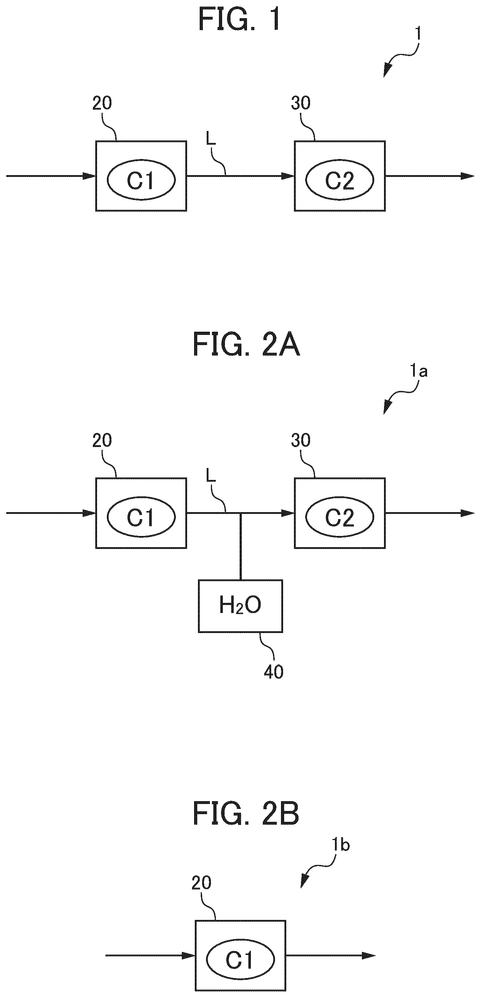

As shown in FIG. 1, the carbon dioxide reduction catalyst device 1 as described in the present embodiment includes a catalytic reactor 20 having a first catalyst C1 and a catalytic reactor 30 having a second catalyst C2. The catalytic reactor 20 and the catalytic reactor 30 are connected by a flow path L, and the catalytic reactor 20 and the catalytic reactor 30 are provided on an upstream side and a downstream side, respectively.

### (First Catalyst)

The first catalyst C1 which is a carbon dioxide reduction catalyst as described in the present embodiment contains Fe (iron) as an essential catalytic metal, and contains at least one of gallium (Ga) or zirconium (Zr). In addition, Na (sodium) is preferably further contained. The carbon dioxide reduction reaction using the first catalyst C1 as described in the present embodiment is a reaction in which a mixed gas of H₂ (hydrogen) and CO₂ (carbon dioxide) is used as a raw material, and hydrocarbons are generated by performing a reverse water gas shift reaction in which CO₂ is reduced to CO (carbon monoxide) and an FT synthesis reaction in which CO is converted into hydrocarbons in one stage. The catalyst as described in the present embodiment contributes to both the reverse water gas shift reaction and the FT synthesis reaction. The carbon dioxide reduction reaction using the catalyst as described in the present embodiment can efficiently produce hydrocarbons having 8 to 16 carbon atoms even under a high flow rate of, for example, a space velocity SV (Space Velocity) = about 5,000 h⁻¹, as compared with conventional FT synthesis reactions.

Fe contained as a catalytic metal in the first catalyst C1 may be in the form of a compound such as an oxide, a carbonate compound, a nitrate compound, a sulfate compound, or the like, and is preferably an oxide. Two or more of these compounds may be contained. Further, Fe is more preferably contained in the catalytic metals as at least any of an Fe-Ga composite oxide containing Fe and Ga or an Fe-Zr composite oxide containing Fe and Zr. By using the catalytic metals containing at least any of the Fe-Ga composite oxide or the Fe-Zr composite oxide, the Fe particles can be further converted into a carbide in the FT synthesis reaction, which promotes a CH₂ growth reaction on the catalyst, promoting growth of carbon chain.

The content of Fe with respect to the catalytic metals in the first catalyst C1 is preferably 55 to 90 mass%, and more preferably 60 to 75 mass%, in terms of metal atoms.

Like Fe, Ga, as a catalytic metal, contained in the first catalyst C1 may be in the form of a compound such as an oxide, a carbonate compound, a nitrate compound, or a sulfate compound, and is preferably an oxide. Two or more of these compounds may be contained. Ga is more preferably contained, as a catalytic metal, in the form of an Fe-Ga composite oxide containing Fe and Ga.

The content of Ga with respect to the catalytic metals in the first catalyst C1 is preferably 10 to 30 mass%, and more preferably 20 to 30 mass%, in terms of metal atoms. When the Ga content is less than 10% by mass, the catalytic metals may not be sufficiently finely micronized. By setting the content of Ga to 30 mass% or less, it is possible to avoid an adverse effect caused by Ga coating a reaction site of Fe, resulting in prevention of a decrease in catalytic activity.

Like Fe, Zr, as a catalytic metal, contained in the first catalyst C1 may be in the form of a compound such as an oxide, a carbonate compound, a nitrate compound, or a sulfate compound, and is preferably an oxide. Two or more of these compounds may be contained. Zr is more preferably contained, as a catalytic metal, in the form of the Fe-Zr composite oxide containing Fe and Zr.

The content of Zr with respect to the catalytic metals is preferably more than 0% by mass and 15% by mass or less, and more preferably 5 to 10% by mass, in terms of metal atoms. By setting the content of Zr to 15 mass% or less, it is possible to avoid an adverse effect due to Zr coating the reaction site of Fe, resulting in prevention of a decrease in catalytic activity.

The catalytic metals of the first catalyst C1 may include both Ga and Zr. When both Ga and Zr are contained as the catalytic metals, these catalytic metals are more preferably contained as the catalytic metals in the form of an Fe-Ga-Zr composite oxide containing Fe, Zr, and Ga. Since the Fe-Ga-Zr composite oxide is more finely micronized as compared with a compound such as an iron oxide, the reaction site of the Fe catalyst increases, whereby the reaction time of the FT synthesis reaction, that is, the time for the carbon chain of the hydrocarbon to be produced growing can be secured.

The first catalyst C1 preferably further contains Na as a catalytic metal. Na functions as a co-catalyst in the catalytic metals and captures CO₂ as Na₂CO₃ to promote a reverse water gas shift reaction in which CO is generated from H₂ and CO₂, thereby improving the CO₂ conversion. Na is preferably present on the surface of the Fe-Zr composite oxide and/or the Fe-Ga-Zr composite oxide in the form of an oxide or the like, separately from the Fe-Zr composite oxide and/or the Fe-Ga-Zr composite oxide. The catalytic metals may contain an alkali metal such as Li, K, Rb, Cs, or the like, instead of Na or together with Na.

The content of Na with respect to the catalytic metals in the first catalyst C1 is preferably 0.5 to 1.5 mass%, and more preferably 1.0 mass%. By setting the content of Na to 0.5% by mass or more, the production efficiency of hydrocarbons having 8 to 16 carbon atoms can be sufficiently improved. Further, by setting the content of Na to 1.5 mass% or less, it is possible to avoid an adverse effect due to Na coating the reaction site of Fe, resulting in prevention of a decrease in catalytic activity.

### (Second Catalyst)

The second catalyst C2 essentially contains Fe (iron) and Co (cobalt) as catalytic metals. In addition, Na (sodium) is preferably further contained. The second catalyst C2 is disposed downstream of the first catalyst C1, and increases the number of carbon atoms of the hydrocarbon produced by the first catalyst C1 to improve the production ratio (yield) of the hydrocarbon having 8 to 16 carbon atoms.

Fe, as a catalytic metal, contained in the second catalyst C2 may be in the form of a compound such as an oxide, a carbonate compound, a nitrate compound, or a sulfate compound, and is preferably an oxide. Two or more of these compounds may be contained. Further, Fe is more preferably contained, as a catalytic metal, in the form of the Fe-Co composite oxide containing Fe and Co. Use of the catalytic metals containing the Fe-Co composite oxide can promote growth of the carbon chain as compared with a compound such as iron oxide, because Co itself has carbon growth reactivity.

The content of Fe with respect to the catalytic metals in the second catalyst C2 is preferably 60 to 90 mass%, and more preferably 70 to 80 mass%, in terms of metal atoms.

Like Fe, Co, as a catalytic metal, contained in the second catalyst C2 may be in the form of a compound such as an oxide, a carbonate compound, a nitrate compound, or a sulfate compound, and is preferably an oxide. Two or more of these compounds may be contained. Co is more preferably contained, as a catalytic metal in the form of an Fe-Co composite oxide containing Fe and Co.

The content of Co, with respect to the catalytic metals in the second catalyst C2 is preferably 10 to 40 mass%, and more preferably 20 to 30 mass%, in terms of metal atoms. By setting the content of Co to 10 mass% or more, the carbon growth reactivity of Co itself can be exhibited. By setting the content of Co to 40 mass% or less, generation of methane as a by-product can be suppressed. Further, the function of reducing carbon dioxide to carbon monoxide in the iron catalyst (reverse water gas shift reaction) can be maintained.

The second catalyst C2 preferably further contains Na as a catalytic metal. Na functions as a co-catalyst in the catalytic metals. Na is preferably present on the surface of the Fe-Co composite oxide in the form of an oxide or the like, separately from the Fe-Co composite oxide. The catalytic metals may include an alkali metal such as Li, K, Rb, Cs, or the like, instead of Na or together with Na.

The content of Na with respect to the catalytic metals in the second catalyst C2 is preferably 0.5 to 1.5 mass%, and more preferably 1.0 mass%. By setting the content of Na to 0.5% by mass or more, the basicity of the iron catalyst can be increased, and the production efficiency of hydrocarbons having 8 to 16 carbon atoms can be sufficiently improved. Further, by setting the content of Na to 1.5 mass% or less, it is possible to avoid an adverse effect caused by Na coating the reaction site of Fe, to suppress the generation of carbon monoxide as a by-product, resulting in prevention of a decrease in catalytic activity.

### (Catalytic Reactor)

The configurations of a catalytic reactor 20 and a catalytic reactor 30 are not particularly limited, and known configurations can be applied. For example, a fixed bed flow reactor having a flow path having a predetermined shape filled with a powdery, particulate, or pelletized catalyst or a carrier supporting the catalyst can be used. The catalytic reactor 20 and the catalytic reactor 30 are independent of each other, and the catalyst temperatures can be set to different temperatures by a temperature raising device (not shown).

### <<Second Embodiment>>

Next, the configuration of the carbon dioxide reduction catalyst device 1a as described in the second embodiment of the present invention will be described with reference to FIG. 2A. The configuration of the carbon dioxide reduction catalyst device 1a is the same as that of the first embodiment except that a water trap 40 is provided in the middle of the flow path L.

### (Water Trap)

The water trap 40 removes water from a fluid supplied to the second catalyst C2. Thereby, in the catalytic reaction using the second catalyst C2, the chemical equilibrium can be shifted in the direction of increasing the number of carbon atoms of the hydrocarbon. Therefore, the yield of the hydrocarbon having 8 to 16 carbon atoms can be improved.

As the water trap 40, a conventionally known water trap can be used. For example, there is a configuration in which a bent pipe is used, condensed water is stored in the bent portion, and condensed water exceeding a certain amount of water is discharged to the outside of the system. In addition to the above, the water trap unit 40 may be configured to trap condensed water by cooling the pipe itself by ice cooling or the like. These may be used alone or as a combination of two or more of them.

### <Carbon Dioxide Reduction Method>

The carbon dioxide reduction method as described in the present embodiment is performed using the above-described carbon dioxide reduction catalyst device 1 or 1a. The carbon dioxide reduction method includes a first catalytic reaction step of bringing a gas containing carbon dioxide into contact with a first catalyst C1 disposed on an upstream side, and a second catalytic reaction step of bringing a gas containing hydrocarbon generated in the first catalytic reaction step into contact with a second catalyst C2 disposed on a downstream side to increase the number of carbon atoms. Between the first catalytic reaction step and the second catalytic reaction step, a step of removing moisture from the hydrocarbon-containing gas generated in the first catalytic reaction step by the water trap 40 may be included.

The catalyst temperature T2 of the second catalyst C2 in the second catalytic reaction step is preferably lower than the catalyst temperature T1 of the first catalyst C1 in the first catalytic reaction step. Thereby, the activity of Co contained in the second catalyst can be controlled, and the yield of hydrocarbons having 8 to 16 carbon atoms can be further improved.

The catalyst temperature T1 is preferably, for example, 340 to 400°C, and the catalyst temperature T2 is preferably, for example, 260 to 340°C.

### <Method for Producing First Catalyst>

The method for producing the first catalyst as described in the present embodiment preferably includes a coprecipitation step and an impregnation step.

### (Coprecipitation Step)

The coprecipitation step is a step of extracting, by a coprecipitation method, a precipitate as a catalyst precursor from an aqueous solution, in which a predetermined amount of a nitrate of Fe and at least any of a nitrate of Ga or a nitrate of Zr is dissolved in distilled water. By the coprecipitation step, the Fe-Ga composite oxide and at least any of the Fe-Zr composite oxide or the Fe-Ga-Zr composite oxide is formed. In the coprecipitation step, it is preferable to obtain a precipitation solution by adding an aqueous urea solution dropwise to the aqueous solution containing Fe and at least any of Ga or Zr. Thereafter, the precipitate is separated from the precipitate solution by filtration, washing or the like, and dried to obtain a precipitate (Fe-Ga composite oxide, Fe-Zr composite oxide, Fe-Ga-Zr composite oxide) as a catalyst precursor.

### (Impregnation Step)

The impregnation step is a step in which an aqueous solution containing Na is added dropwise to the precipitate obtained in the coprecipitation step, followed by drying for a predetermined time, and the obtained powder is calcined at a predetermined temperature. The impregnation step allows the Na compound to be more heavily distributed closer to the surface of the composite oxide. Examples of the aqueous solution containing Na include an aqueous solution of NaNO₃. The NaNO₃ aqueous solution can be added dropwise under ultrasonic vibration. This allows the Na compound to exist closer to the surface of the composite oxide and be uniformly distributed in a direction across the surface of the composite oxide. The calcination temperature may be, for example, 550°C, and the calcination time may be 4 hours.

### <Method for Producing Second Catalyst>

The method for producing the second catalyst as described in the present embodiment preferably includes a hydrothermal synthesis step and an impregnation step.

### (Hydrothermal Synthesis Step)

The hydrothermal synthesis step is a step of extracting a precipitate as a catalyst precursor by a hydrothermal synthesis method from an aqueous solution in which a predetermined amount of a nitrate of Fe and a predetermined amount of a nitrate of Co are dissolved in an aqueous urea solution. A Fe-Co composite oxide is formed by the hydrothermal synthesis step. In the hydrothermal synthesis step, the aqueous solution containing Fe and Co is preferably subjected to a hydrothermal synthesis step using an autoclave to obtain a precipitate solution. Thereafter, the precipitate is separated from the precipitate solution by filtration, washing, or the like, and dried to obtain a precipitate (Fe-Co composite oxide), which is a catalyst precursor.

### (Impregnation Step)

The impregnation step may be the same as the impregnation step in the first catalyst production method.

The present invention is not limited to the above-described embodiments, and modifications and improvements within a range capable of achieving the object of the present invention are included in the present invention.

### EXAMPLES

Next, examples of the present invention will be described, but the present invention is not limited to these examples.

### <Example 1>

### [Preparation of First Catalyst]

A nitrate of Fe (Fe(NO₃)₃·9H₂O) as a catalytic metal of the first catalyst, a nitrate of Zr (ZrO(NO₃)₂·2H₂O) similarly as a catalytic metal, and a nitrate of Ga (Ga(NO₃)₃·6H₂O) similarly as a catalytic metal were weighed so that the mass ratio of Fe:Zr:Ga in terms of metal atoms was 6:1:3, and dissolved in distilled water. Next, while stirring the aqueous solution, an aqueous CH₄N₂O solution was added dropwise at a rate of 2 ml/min, and the pH was fixed at 8.5 to obtain a precipitate solution containing Fe, Zr, and Ga as precipitates. Then, the precipitate solution was aged at room temperature for 24 hours, and a precipitate was separated by repeating filtration and washing. The separated precipitate was dried at 60°C for 12 hours to obtain a Fe-Ga-Zr catalyst precursor.

An aqueous NaNO₃ solution was added dropwise to the Fe-Ga-Zr catalyst precursor under ultrasonic vibration at 92 kHz so that the Na content was 1.0 mass%. Then, the mixture was dried under vacuum of 5,000 Pa for 1 hour, and further dried at 60°C under normal atmospheric pressure for 12 hours to obtain a powder. The obtained powder was calcined at 550°C for 4 hours to obtain a first catalyst of Example 1.

### [Preparation of Second Catalyst]

A nitrate of Fe (Fe(NO₃)₃·9H₂O) as a catalytic metal of the second catalyst and a nitrate of Co (Co(NO₃)₂·6H₂O) similarly as a catalytic metal were weighed so that the mass ratio of Fe:Co in terms of metal atoms was 3:1, and dissolved in urea water. Next, the aqueous solution was stirred for 1 hour, and then transferred to an autoclave vessel and subjected to hydrothermal synthesis at 120°C for 12 hours to obtain a precipitate solution containing Fe and Co as precipitates. Then, the precipitate solution was aged at room temperature for 24 hours, and a precipitate was separated by repeating filtration and washing. The separated precipitate was dried at 60°C for 12 hours to obtain a Fe-Co catalyst precursor.

An aqueous NaNO₃ solution was added dropwise to the Fe-Co catalyst precursor under ultrasonic vibration at 92 kHz so that the Na content was 1.0 mass%. Then, the mixture was dried under vacuum of 5,000 Pa for 1 hour, and further dried at 60°C under normal pressure for 12 hours to obtain a powder. The obtained powder was calcined at 550°C for 4 hours to obtain a second catalyst of Example 1.

### [Preparation of Carbon Dioxide Reduction Catalyst Device]

A carbon dioxide reduction catalyst device 1 shown in FIG. 1 was manufactured using the first catalyst and the second catalyst obtained as described above. As the catalytic reactor, a fixed bed flow reactor was used, and 0.25 g of the first catalyst in the form of pellets of 0.4 to 0.8 mm square was used. The pellets were loaded in a reaction tube (inner diameter: 6 mm) at a length of 5 cm and used. The second catalyst was also used in the same shape, weight, and loading as the first catalyst. For the reaction temperature, the catalyst temperature T1 of the first catalyst was set to 380°C, and the catalyst temperature T2 of the second catalyst was set to 300°C.

### <Example 2>

This example was the same as Example 1 except that a carbon dioxide reduction catalyst device 1a shown in FIG. 2A was manufactured.

### <Example 3>

This example was the same as Example 2 except that the catalyst temperature T2 of the second catalyst was set to 220°C.

### <Example 4>

This example was the same as Example 2 except that the catalyst temperature T2 of the second catalyst was set to 260°C.

### <Example 5>

This example was the same as Example 2 except that the catalyst temperature T2 of the second catalyst was set to 340°C.

### <Example 6>

This example was the same as Example 2 except that the second catalyst was prepared so that the Na content (addition amount) of the second catalyst was 0.5% by mass.

### <Example 7>

This example was the same as Example 2 except that the second catalyst was prepared so that the Na content (addition amount) of the second catalyst was 1.5 mass%.

### <Example 8>

This example was the same as Example 2 except that the second catalyst was prepared so that the Co content (addition amount) of the second catalyst was 10 mass% (the second catalyst was prepared so that the Fe content was 89 mass% and the Na content was 1.0 mass%).

### <Example 9>

This example was the same as Example 2 except that the second catalyst was prepared so that the Co content (addition amount) of the second catalyst was 25 mass% (the second catalyst was prepared so that the Fe content was 74 mass% and the Na content was 1.0 mass%).

### <Example 10>

The procedure of this example was the same as in Example 2 except that the second catalyst was prepared so that the Co content (addition amount) of the second catalyst was 40 mass% (the second catalyst was prepared so that the Fe content was 59 mass% and the Na content was 1.0 mass%).

### <Comparative Example 1>

The procedure was the same as in Example 1 except that a carbon dioxide reduction catalyst device 1b shown in FIG. 2B was manufactured using only the first catalyst obtained as described above. As shown in FIG. 2B, the carbon dioxide reduction catalyst device 1b includes only the catalytic reactor 20 having the first catalyst C1 as the catalytic reactor. The catalyst temperature T1 of the first catalyst was set to 380°C.

### <Comparative Example 2>

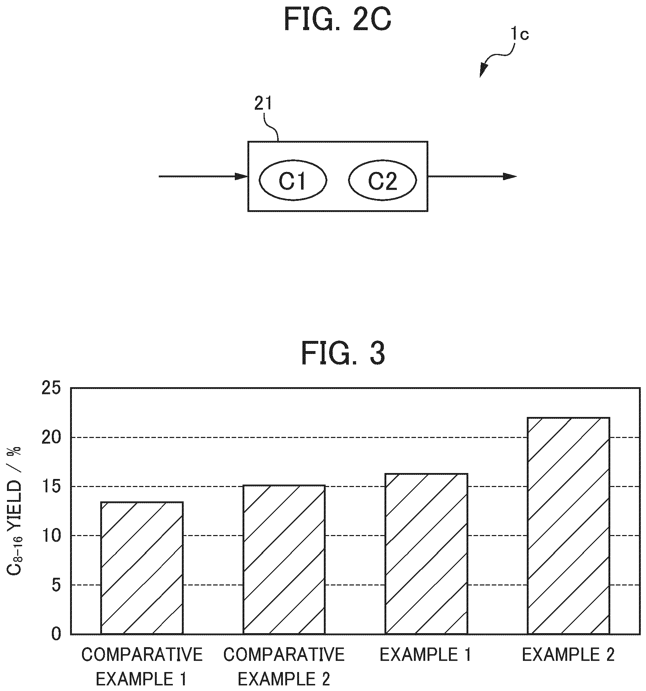

The procedure was the same as in Example 1 except that a carbon dioxide reduction catalyst device 1c shown in FIG. 2C was manufactured using the first catalyst and the second catalyst obtained as described above. In the carbon dioxide reduction catalyst device 1c, as shown in FIG. 2C, the first catalyst C1 and the second catalyst C2 were loaded into the same catalyst reactor 21 and adjusted to the same catalyst temperature. The catalyst temperature T1 of the first catalyst and the catalyst temperature T2 of the second catalyst were set to 380°C.

### <Comparative Example 3>

The procedure was the same as in Example 2 except that the second catalyst was prepared without adding Na to the second catalyst.

### <Comparative Example 4>

The procedure was the same as in Example 2 except that the second catalyst was prepared without adding Co to the second catalyst.

### [Evaluation]

A carbon dioxide reduction reaction was carried out by the following method using each of the carbon dioxide reduction catalyst devices of the Examples and Comparative Examples. The reaction gases were 0.28 NL/h of CO₂ and 0.84 NL/h of H₂ (CO₂/H₂ = 1/3). The W/F (catalyst weight/gas flow rate) and the space velocity (SV) were set to 5.0 g·h/mol and 5,000 h⁻¹, respectively. The pressure was 3 MPa, and the reaction time was 4 hours. The gas component after the catalytic reaction was qualitatively and quantitatively analyzed by on-line gas chromatography (Shimadzu, GC-2014AT, Detector: thermal conductivity detector (TCD)) and a flame ionization detector (FID) (Shimadzu, GC-2014AF). The liquid component after the catalytic reaction was also qualitatively and quantitatively analyzed by off-line gas chromatography (Shimadzu, GC-2014AF, Detector: flame ionization detector (FID)).

### (CO₂ conversion)

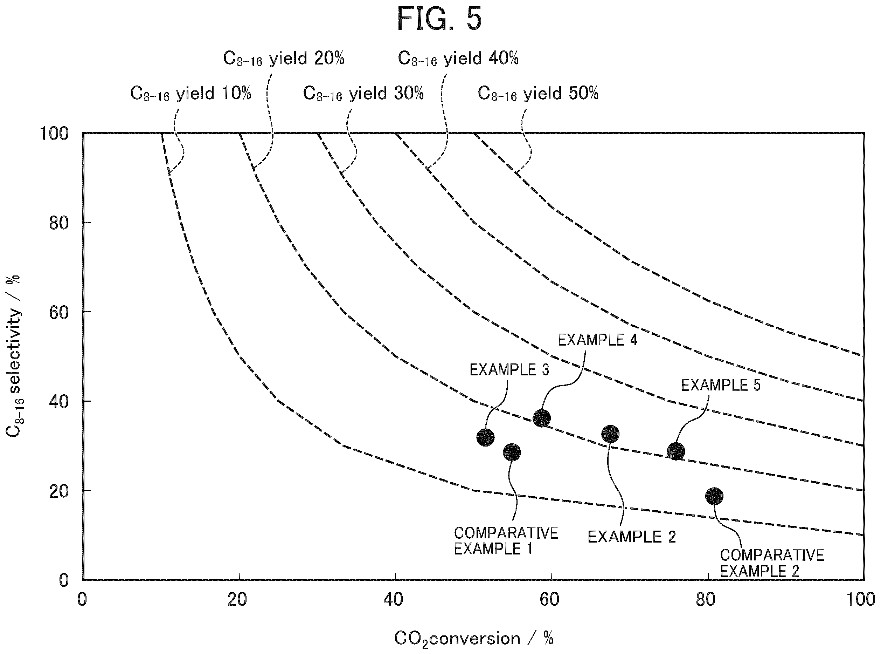

The conversion of CO₂ by the carbon dioxide reduction reaction was determined by the following formula (1). The results are shown in FIGs. 5, 6A, and 7A. CO₂ conversion (%) = ((CO₂ concentration before reaction) - (CO₂ concentration after reaction))/(CO₂ concentration before reaction) × 100 ··· (1)

### (C₈₋₁₆ selectivity)

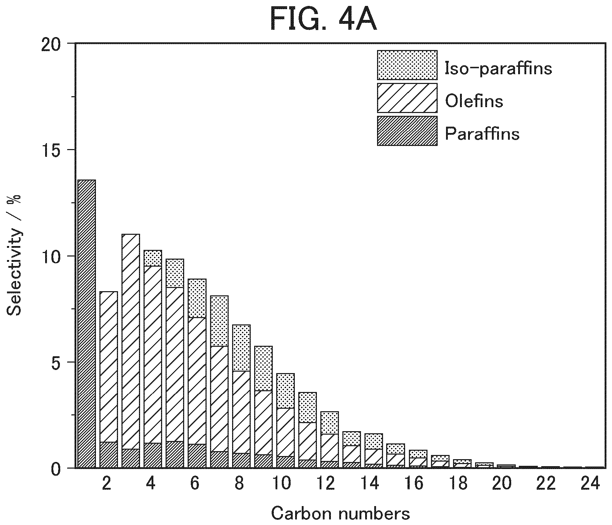

The selectivity of the hydrocarbon (C₈₋₁₆) having 8 to 16 carbon atoms produced by the carbon dioxide reduction reaction was determined by the following formula (2). The results are shown in FIGs. 5, 6B, and 7B. The selectivity of the hydrocarbon having each carbon number and structure was determined in the same manner as in the following formula (2). The results are shown in FIGs. 4A to 4C. C₈₋₁₆ selectivity (%) = (C₈₋₁₆-containing component concentration)/((CO₂ concentration before reaction)-(CO₂ concentration after reaction)) × 100 ··· (2)

### (C₈₋₁₆ Production Ratio (Yield))

The production ratio of the hydrocarbon (C₈₋₁₆) having 8 to 16 carbon atoms produced by the carbon dioxide reduction reaction was determined by the following formula (3). The results are shown in FIGs. 3, 5, 6C, and 7C. C₈₋₁₆ production ratio (%) = CO₂ conversion × C₈₋₁₆ selectivity/100 ··· (3)

As shown in FIG. 3, it is clear that the carbon dioxide reduction catalyst device as described in each of the Examples had a higher C₈₋₁₆ production ratio (yield) than the carbon dioxide reduction catalyst device as described in each of the Comparative Examples.

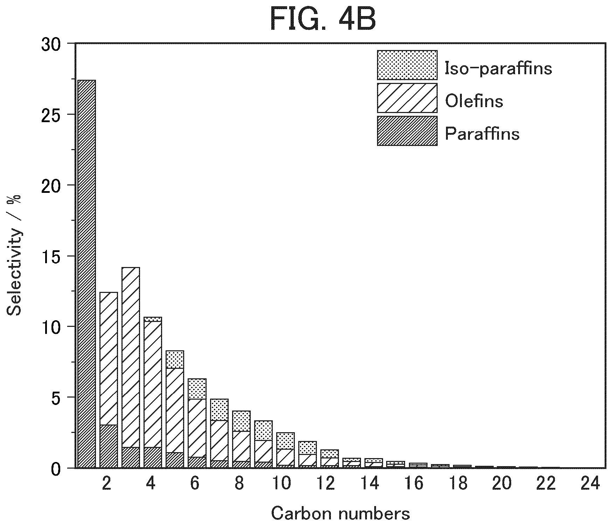

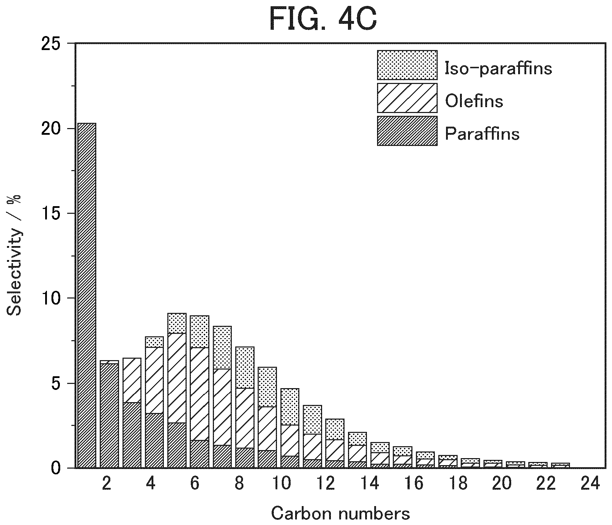

FIG. 4A is a graph showing the number of carbon atoms and the selectivity of the hydrocarbons, which were produced by the carbon dioxide reduction catalyst device as described in Comparative Example 1. Similarly, FIG. 4B is a graph corresponding to Comparative Example 2, and FIG. 4C is a graph corresponding to Example 1. The term "paraffins" in FIGs. 4A to 4C means saturated chain hydrocarbons having a linear structure. The term "iso-paraffins" means a saturated chain hydrocarbon having a branched structure. The term "olefins" means a chain hydrocarbon having a double bond.

As shown in FIG. 4A, the carbon dioxide reduction catalyst device of Comparative Example 1, in which only the first catalyst was used, had a C₈₋₁₆ production ratio of 15% or less. Further, as shown in FIG. 4B, in the carbon dioxide reduction catalyst device of Comparative Example 2 in which the first catalyst and the second catalyst were used at the same catalyst temperature, although the C₈₋₁₆ production ratio was slightly improved as compared with Comparative Example 1, the production ratio of hydrocarbons having a small number of carbon atoms such as methane was high. As shown in FIG. 4C, in the carbon dioxide reduction catalyst device of Example 1, in which the catalyst temperature of the first catalyst was set higher than the catalyst temperature of the second catalyst, the C₈₋₁₆ production ratio was further improved as compared with Comparative Example 2, and the production ratio of hydrocarbons having a small number of carbon atoms such as methane was reduced.

FIG. 5 is a chart showing a relationship between C₈₋₁₆ selectivity and CO₂ conversion of each of the Examples and the Comparative Examples and a relationship with C₈₋₁₆ production ratio (yield) correlated with these results. As shown in FIG. 5, by setting the catalyst temperature of the first catalyst to 380°C and the catalyst temperature of the second catalyst to 260°C to 340°C, the C₈₋₁₆ production ratio (yield) of 20% or more could be achieved.

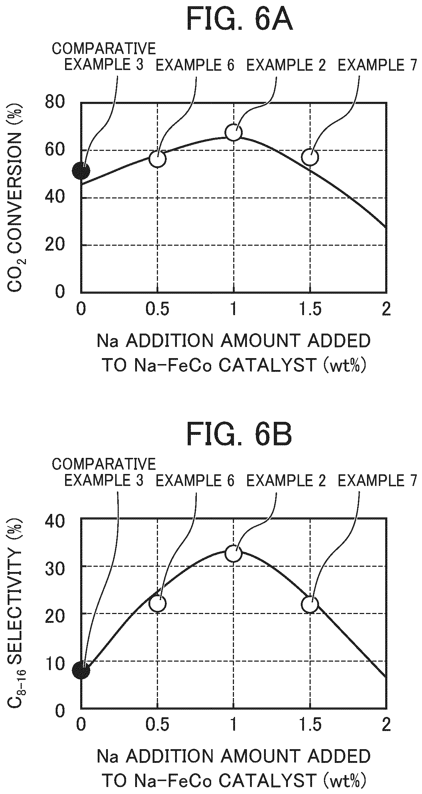

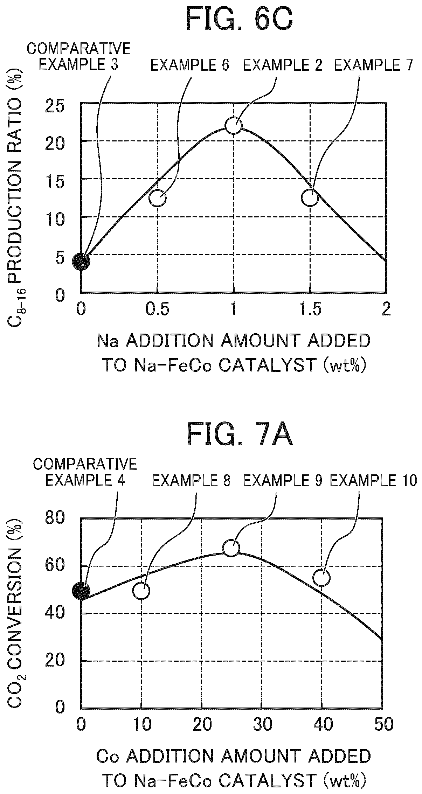

FIGs. 6A, 6B, and 6C are graphs comparing the CO₂ conversion, the C₈₋₁₆ selectivity, and the C₈₋₁₆ production ratio (yield) when only the Na addition amount of the second catalyst was changed under the same conditions. From the results of FIGs. 6A, 6B, and 6C, it is assumed that the C₈₋₁₆ production activity improved as the Na addition amount was increased, but when the Na addition amount was increased by 1.0% by mass or more, since Na covered the reaction site of the Fe catalyst, the activity monotonically decreased. Therefore, it is clear that the Na addition amount is preferably in the range of 0.5 to 1.5 mass%. In particular, it is clear that Example 2 in which the Na addition amount was 1.0 mass% was most preferable because it is excellent in all results.

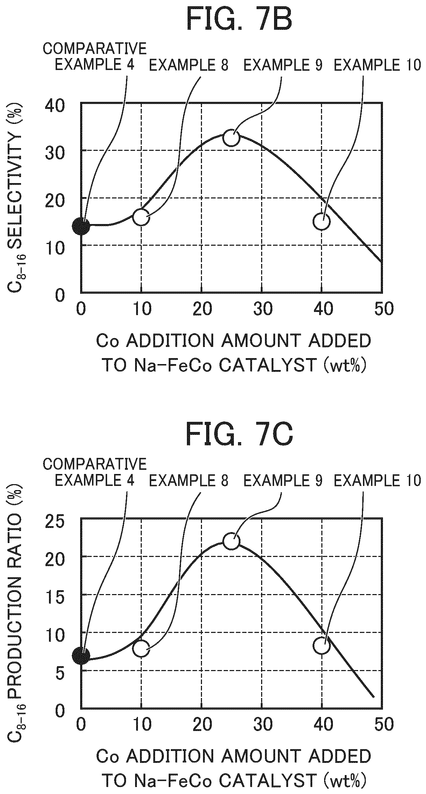

FIGs. 7A, 7B, and 7C are graphs comparing the CO₂ conversion, the C₈₋₁₆ selectivity, and the C₈₋₁₆ production ratio (yield) when only the Co addition amount of the second catalyst was changed under the same conditions. As the Co addition amount was increased, the C₈₋₁₆ production activity improved, but when the Co addition amount was increased by 25% by mass or more, since Co covers the reaction site of the Fe catalyst, the activity monotonically decreased and methane was produced as a by-product. Therefore, it is clear that the Co addition amount is preferably in the range of 10 to 40% by mass. In particular, it is clear that Example 9 in which the Co addition amount was 25 mass% was most preferable because it is excellent in all results.

### EXPLANATION OF REFERENCE NUMERALS

- 1, 1a: Carbon dioxide reduction catalyst device
- C1: First catalyst
- C2: Second catalyst
- 40: Water trap

## Claims

1. A carbon dioxide reduction catalyst device for reducing carbon dioxide by a hydrogenation reaction of carbon dioxide to produce hydrocarbons,
the device comprising: a first catalyst containing Fe and at least one of Ga or Zr as catalytic metals; and
a second catalyst containing Fe and Co as catalytic metals,
wherein the second catalyst is disposed downstream of the first catalyst.

2. The carbon dioxide reduction catalyst device according to claim 1, wherein at least one of the first catalyst or the second catalyst further contains Na as a catalytic metal.

3. The carbon dioxide reduction catalyst device according to claim 2, wherein at least one of the first catalyst or the second catalyst contains Na in an amount that accounts for 0.5 to 1.5 mass% of the total mass of catalytic metals.

4. The carbon dioxide reduction catalyst device according to claim 1 or 2, wherein the second catalyst contains Co in an amount that accounts for 10 to 40 mass% of the total mass of the catalytic metals.

5. The carbon dioxide reduction catalyst device according to claim 1 or 2, comprising a water trap between the first catalyst and the second catalyst.

6. The carbon dioxide reduction catalyst device according to claim 1 or 2, wherein the first catalyst contains at least one of an Fe-Ga composite oxide containing Fe and Ga or an Fe-Zr composite oxide containing Fe and Zr.

7. A method of reducing carbon dioxide, the method comprising using the carbon dioxide reduction catalyst device according to claim 1,
wherein a catalyst temperature T2 of the second catalyst is lower than a catalyst temperature T1 of the first catalyst.

8. The carbon dioxide reduction according to claim 7, wherein the catalyst temperature T2 is in a range of 260°C to 340°C.

9. A method for producing the first catalyst according to claim **1,** comprising
a coprecipitation step of extracting a precipitate by a coprecipitation method from an aqueous solution in which a predetermined amount of at least one of a nitrate of Fe, a nitrate of Ga, or a nitrate of Zr is dissolved in distilled water.

10. The method for producing a catalyst according to claim 9, further comprising, after the coprecipitation step, an impregnation step of adding an aqueous solution containing Na to the precipitate dropwise, followed by drying for a predetermined period of time, and calcining the obtained powder at a predetermined temperature.

11. The method for producing a catalyst according to claim 9 or 10, wherein in the coprecipitation step, a precipitation solution is obtained by adding an aqueous urea solution dropwise to an aqueous solution in which a predetermined amount of at least one of the nitrate of Fe, the nitrate of Ga, or the nitrate of Zr is dissolved in distilled water.

## Amended claims

### Amended claims under Art. 19.1 PCT

1. (Original) A carbon dioxide reduction catalyst device for reducing carbon dioxide by a hydrogenation reaction of carbon dioxide to produce hydrocarbons,
the device comprising: a first catalyst containing Fe and at least one of Ga or Zr as catalytic metals; and
a second catalyst containing Fe and Co as catalytic metals,
wherein the second catalyst is disposed downstream of the first catalyst.

2. (Original) The carbon dioxide reduction catalyst device according to claim 1, wherein at least one of the first catalyst or the second catalyst further contains Na as a catalytic metal.

3. (Original) The carbon dioxide reduction catalyst device according to claim 2, wherein at least one of the first catalyst or the second catalyst contains Na in an amount that accounts for 0.5 to 1.5 mass% of the total mass of catalytic metals.

4. (Original) The carbon dioxide reduction catalyst device according to claim 1 or 2, wherein the second catalyst contains Co in an amount that accounts for 10 to 40 mass% of the total mass of the catalytic metals.

5. (Original) The carbon dioxide reduction catalyst device according to claim 1 or 2, comprising a water trap between the first catalyst and the second catalyst.

6. (Original) The carbon dioxide reduction catalyst device according to claim 1 or 2, wherein the first catalyst contains at least one of an Fe-Ga composite oxide containing Fe and Ga or an Fe-Zr composite oxide containing Fe and Zr.

7. (Original) A method of reducing carbon dioxide, the method comprising using the carbon dioxide reduction catalyst device according to claim 1,
wherein a catalyst temperature T2 of the second catalyst is lower than a catalyst temperature T1 of the first catalyst.

8. (Original) The carbon dioxide reduction according to claim 7, wherein the catalyst temperature T2 is in a range of 260°C to 340°C.

9. (Amended) A method for producing the first catalyst according to claim 1, comprising
a coprecipitation step of extracting a precipitate by a coprecipitation method from an aqueous solution in which a predetermined amount of at least one of a nitrate of Fe, a nitrate of Ga, or a nitrate of Zr is dissolved in distilled water,
wherein in the coprecipitation step, a Fe-Ga-Zr catalyst precursor is obtained by adding an aqueous urea solution dropwise to an aqueous solution in which a predetermined amount of at least one of the nitrate of Fe, the nitrate of Ga, or the nitrate of Zr is dissolved in distilled water, to separate the precipitate from a resulting precipitate solution.

10. (Original) The method for producing a catalyst according to claim 9, further comprising, after the coprecipitation step, an impregnation step of adding an aqueous solution containing Na to the precipitate dropwise, followed by drying for a predetermined period of time, and calcining the obtained powder at a predetermined temperature.

11. (Canceled)
